(19) **Europäisches Patentamt**
**European Patent Office**
**Office européen des brevets**

(11) **EP 3 502 687 B1**

(12) **EUROPEAN PATENT SPECIFICATION**

(45) Date of publication and mention
of the grant of the patent:
**29.06.2022 Bulletin 2022/26**

(21) Application number: **17208745.4**

(22) Date of filing: **20.12.2017**

(51) International Patent Classification (IPC):
***G01N 33/22*** *(2006.01)*     ***G01N 25/18*** *(2006.01)*
*G01N 27/18* *(2006.01)*

(52) Cooperative Patent Classification (CPC):
**G01N 33/225; G01N 25/18;** G01N 25/005;
G01N 27/18

(54) **DETERMINATION OF GAS PARAMETERS**

BESTIMMUNG VON GASPARAMETERN

DÉTERMINATION DE PARAMÈTRES DE GAZ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**26.06.2019 Bulletin 2019/26**

(73) Proprietor: **Sensirion AG**
**8712 Stäfa (CH)**

(72) Inventors:
• **HORNUNG, Mark**
**8712 Stäfa (CH)**

• **RÜEGG, Andreas**
**8712 Stäfa (CH)**
• **KILIANI, David**
**8712 Stäfa (CH)**

(74) Representative: **Piticco, Lorena et al**
**Isler & Pedrazzini AG**
**Giesshübelstrasse 45**
**Postfach 1772**
**8027 Zürich (CH)**

(56) References cited:
**WO-A1-2015/075278**    **US-A1- 2010 154 510**
**US-A1- 2011 126 611**    **US-A1- 2016 138 951**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a method, a thermal sensor device/system, and a computer program product for determining a parameter of a gas, for instance, a Prandtl number or a combustion parameter such as a calorific value (heat of combustion per unit mass or per unit volume), a Wobbe index, or a methane number.

PRIOR ART

It is known to use correlation functions for calculating combustion parameters of a gas based on measured heat transfer and volumetric heat capacity parameters. It is also known that the accuracy of determining such combustion parameters may be substantially improved by using an independent third gas parameter.

DE 101 22 039 A1 teaches to guide a gas with a constant volumetric flow through a measurement tube. A first temperature sensor, a second temperature sensor, and a heater element therebetween are provided on the measurement tube. A temperature difference between the temperature sensors is monitored while the heater element is operated at constant power. A calorific value of the gas is determined as a linear function of the temperature difference by using empirically determined coefficients. This method has the drawback of being rather inaccurate.

EP 2 015 056 discloses a system that uses a thermal flow sensor and a sonic nozzle for determining a combustion parameter. The sonic nozzle creates, however, a considerable pressure drop in the gas.

US 2016/0138951 A1 discloses a method and measuring apparatus for determining specific quantities for the gas quality in which the gas or gas mixture flows through an ultrasonic flow sensor and a microthermal sensor. The sound velocity, the thermal conductivity and the thermal capacity derived from said sensors are subsequently used for the determination of specific quantities for the gas quality.

US 2010/0154510 A1 discloses a method for sensing gas composition based on thermal constants of a variable electrical resistor. The thermal response of the resistor is determined by the thermal conductivity and thermal capacity of the surrounding gas.

From US 2011/0126611 A1 a hydrogen sensor is known, which includes a support, temperature sensors, a heating element and an evaluation unit. The evaluation unit determines a hydrogen concentration based on temperatures detected by the temperature sensors.

SUMMARY OF THE INVENTION

It is an object of the present invention to provide the possibility to better determine gas parameters.

This object is achieved by the method according to claim 1. According thereto, a method for determining a gas parameter of a gas of interest is suggested. The gas parameter may be a Prandtl number, a hydrogen concentration, or a combustion parameter of the gas. The gas may preferably be a natural gas. The method comprises the following steps:

- operating a thermal sensor device being exposed to said gas and comprising at least one temperature sensor, preferably two temperature sensors, and at least one heater element;
- calculating from an output of said thermal sensor device, i.e. from at least the temperature readings, in some embodiment also from further readings such as an absolute pressure reading:

  (i) a temperature T of said gas;
  (ii) a heat-transfer parameter of said gas, wherein said heat-transfer parameter of said gas is determined from a stationary temperature distribution in said gas caused by said at least one heater element;
  (iii) a heat-capacity related parameter of said gas, wherein said heat-capacity related parameter of said gas is determined from a time-varying heating scheme applied to said heater element;
  (iv) a natural-convection parameter $\frac{1}{vD}$ of said gas related to a natural convection caused in said gas by action of said at least one heater element.

Then, said gas parameter is calculated from said heat-transfer parameter, said heat-capacity related parameter, and said natural-convection parameter. This may be done, for example, as shown below.

The temperature of the gas may be a simple reading of the at least one temperature sensor or of a further temperature sensor included to the thermal sensor device. The heat-transfer parameter may be the thermal conductivity $\lambda$ of the gas, the heat-capacity related parameter may be the thermal diffusivity $D$ or the volumetric heat capacity $c_p\rho$ of

the gas. These parameters may be measured as taught, for example, in US 6,019,505.

In the context of the present invention, the term *"natural-convection parameter related to a natural convection caused in said gas by action of said at least one heater element"* is to be understood as a parameter that characterizes the convection behaviour of the gas of interest under action of said at least one heater element as measured by the at least one temperature and, in some embodiments, additionally by using said heater element as a sensor, in other embodiments that comprise more than one temperature sensor, by using the temperature reading of said further temperature sensor. The natural-convection parameter of the gas may be defined as $\frac{1}{\nu D} = \frac{c_p \rho^2}{\lambda \eta}$, wherein $\nu = \frac{\eta}{\rho}$ is the kinematic viscosity of the gas, $\eta$ the dynamic viscosity of the gas, $c_p$ the specific heat capacity at constant pressure of the gas, $p$ the density of the gas, $\lambda$ its thermal conductivity, and $D$ its thermal diffusivity.

The present invention is based on the insight that gas parameters such as the Prandtl number, the hydrogen concentration, or the combustion parameters *(i.e.* parameters that characterize the combustion behavior of the gas) of many gases, including natural gases, can be calculated if the heat-transfer parameter *(i.e.* a parameter that characterizes the heat transfer behavior of the gas, *e.g.,* the thermal conductivity of the gas) and the heat-capacity related parameter *(i.e.* the specific heat capacity per unit mass or unit volume or the thermal diffusivity of the gas) are measured, and if additionally the natural-convection parameter *(i.e.* a parameter that characterizes currents in a gas due to temperature or pressure differences in said gas) is measured. The natural convection may be determined by means of the same sensor arrangement by means of which the further independent gas parameters (heat-transfer parameter and heat-capacity related parameter) are measured. From the heat-transfer parameter, the heat-capacity related parameter, and the natural convection parameter and a readily available temperature of the gas, one could say from the ambient temperature, a gas parameter such as the Prandtl number, the hydrogen concentration, or the combustion parameter of the gas may be calculated.

The present invention dispenses therefore with the need for establishing a considerably pressure drop in the gas, *e.g.* by using a sonic nozzle. The present invention allows using, for example, a single thermal sensor *(e.g.* a thermal flow sensor) to determine, by operation under an appropriately configured control device, the gas parameter. Accordingly, it is possible to determine the gas parameter in a simple and cost-efficient manner. The present invention allows designing a compact and cost-efficient device. Moreover, no particular pressure or finite flow conditions have to be established. It is sufficient to establish a zero-flow condition, preferably at constant heating power from the heater element, for generating the natural convection phenomenon, and reading-out the temperature signals for the calculation of the natural-convection parameter and to measure heat-transfer parameter and heat-capacity related parameter in a known manner. The present invention allows determining the gas parameters online, *i.e.* in real-time.

In some embodiments, said natural-convection parameter $\frac{1}{\nu D}$ is calculated from said heat-transfer parameter and said temperature of said gas. A preferred way of calculation uses a polynomial expansion of said output of said thermal sensor device related to the natural-convection parameter measurement, the expansion being in said natural-convection parameter $\frac{1}{\nu D}$, wherein expansion terms are preferably empirically determined functions of said heat-transfer parameter and said temperature T of said gas. Other expansions may be used.

As a preferred embodiment, the following calculation is suggested.

First, the heat-transfer parameter such as the thermal conductivity $\lambda$ and the heat-capacity related parameter such as the thermal diffusivity D or the volumetric heat capacity $c_p\rho$ are determined by means of said thermal sensor device.

The thermal conductivity $\lambda$ may be determined and used as said heat-transfer parameter. The thermal conductivity $\lambda$ may be measured as described in US 6,019,505. Preferably, $\lambda$ is determined by measuring a stationary temperature distribution across the thermal sensor device due to the heater element action. The maximum temperature of the thermal sensor device is an inverse function of the thermal conductivity $\lambda$ of the surrounding gas. Accordingly, the maximum temperature measured by the temperature sensor of the device with the heater element at a specific, e.g. a maximum heating power, may be used to determine the thermal conductivity $\lambda$. The inverse function may be determined beforehand by means of calibration measurements and implemented by the devices electronic means, e.g. by means of a lookup table.

The thermal diffusivity D may be determined and used as said heat-capacity related parameter. Alternatively, volumetric heat capacity $c_p\rho$ of said gas may be determined, from which the thermal diffusivity may be calculated as

$$D = \frac{\lambda}{c_p\rho}$$

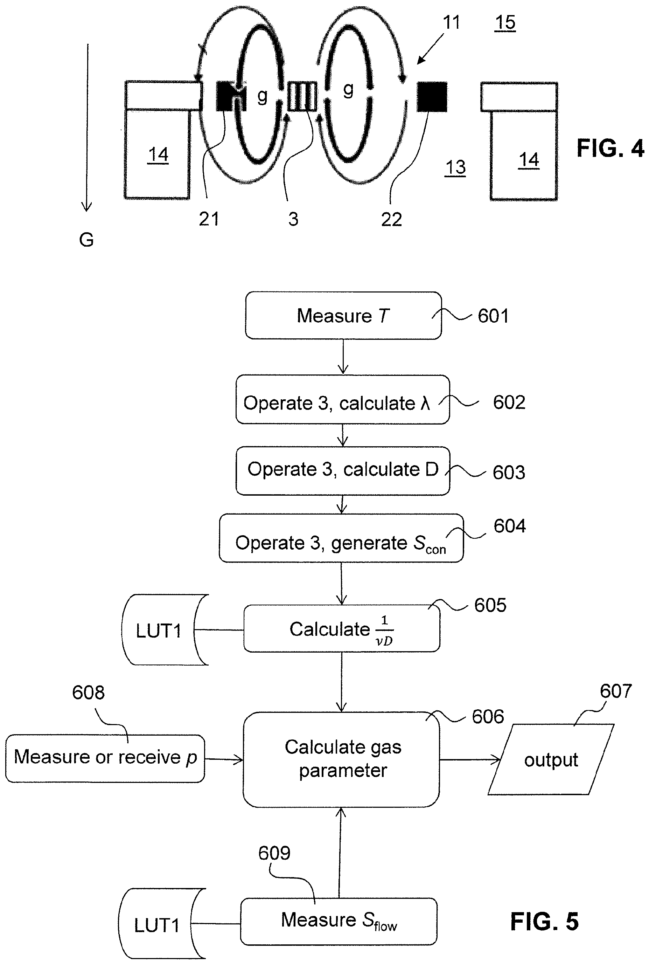

. The thermal diffusivity $D$ may be measured as described in US 6,019,505. Preferably, $D$ is determined by activating the at least one heater element in a heat-wave scheme, e.g. by increasing and decreasing the heating power in a sinusoidal manner. Accordingly, D may be determined from a time-varying heating scheme applied to said heater element. At least one temperature sensor spaced at a certain distance measures the propagation of a heat wave generated by means of the heater element. The phase difference between the generated heat wave at the heater element and the at least one temperature sensor is a function of thermal diffusivity D. Preferably, instead of measuring the phase difference between the heater element and the one temperature sensor, two temperature sensors with different spacing to the heater element are used for measuring the diffusivity D *(e.g.* configuration as in Fig. 4). In the two temperature-sensor configuration, the phase difference of the generated heat wave at the two temperature sensors (as shown e.g. in Fig. 4) is a function of the thermal diffusivity D. This function may be determined by means of calibration measurements. This function may be determined beforehand by means of calibration measurements and implemented by the devices electronic means, e.g. by means of a lookup table.

Next, a zero gas-flow situation in the thermal sensor device may be established (i.e. no flow through the thermal sensor due to, for example, any pressure difference between gas inlet and outlet of said thermal sensor device or, in other words, without action of the heater element, no directed flows such as convection flows occur in the gas). Then, the heater element may be set to heat, preferably with constant heating power, to create the natural convection in the gas, around the heater. The temperature sensors are arranged to be exposed to the convection flows, whereby the convection may be evaluated. It is conceivable to use a varying heater power. The natural-convection parameter related signal $S_{con}$, *i.e.* the thermal sensor output under these conditions *(e.g.* at temperature difference or sum of the two temperature sensors), is read out from the at least one temperature sensor, preferably from two temperature sensors, the signal being the difference. It is also possible to include the heater element into the generation of the output (e.g. resistivity measurement). The thermal sensor output may be, for example, a temperature difference between two temperature sensors exposed to the natural convection, preferably sampling different locations in the convection flows at which different temperatures are present.

The natural-convection parameter related signal $S_{con}$ may be written in a polynomial expansion of the natural-convection parameter $\frac{1}{vD} = \frac{c_p \rho^2}{\lambda \eta}$ as follows:

$$S_{con} = a + b\left(\frac{1}{vD}\right) + c\left(\frac{1}{vD}\right)^2 + \cdots$$

wherein expansion coefficients *a, b, c* etc. are functions of the thermal conductivity $\lambda$ and the gas temperature *T.* The coefficients *a, b, c* etc. may be determined beforehand by means of calibration measurement and may be stored and made available to the device by means of lookup tables or models or otherwise.

The thermal conductivity $\lambda$, the gas temperature T, as determined by measurement, and the expansion coefficients *a, b, c* etc. may therefore be used for deducing from the natural-convection related signal $S_{con}$ of the thermal sensor device said natural-convection parameter $\frac{1}{vD}$. One may use a linear expansion or a quadratic expansion, form which follows, respectively:

- linear expansion: $\frac{1}{vD} = \frac{(S_{con} - a)}{b}$ ; and

- quadratic expansion: $\frac{1}{vD} = \frac{1}{2c}\left(-b + \sqrt{b^2 - 4c(a - S_{con})}\right)$ .

Higher terms may be considered but for many cases, linear or quadratic expansion is sufficient. These functions may be built into the control device for calculation of $\frac{1}{vD}$ from $S_{con}$.

From the natural-convection parameter $\frac{1}{vD}$ and the thermal diffusivity D one readily obtains, for example, the Prandtl number $Pr = \frac{v}{D} = \frac{1}{D^2\left(\frac{1}{vD}\right)}$. From the Prandtl number one may estimate, e.g. by means of one or more lookup table that stores a beforehand established correlation of the Prandtl number Pr and the hydrogen concentration, a hydrogen concentration of a natural gas of interest.

It is to be understood that these three parameters $\left(\lambda, D, \frac{1}{vD}\right)$ may be combined together to a new set of parameters such as, for example: *(λ, c_pρ, Pr)*, wherein $Pr = \frac{c_p \eta}{\lambda}$ is the Prandtl number.

In some embodiments, said thermal sensor device comprises, as said at least one temperature sensor, a first temperature sensor and a second temperature sensor, said heater element being arranged therebetween. This is the design of a known thermal flow sensor, *e.g.* on a membrane or bridge spanning a substrate cavity. By using at least two temperature sensors, one does not need to read out heater element related parameters (such as its resistance) for determining a temperature value but one simply uses the two temperature readings and uses, for example, the difference or sum as $S_{con}$. Such a differential measurement is advantageous as the device degradation is reduced. Moreover, the heater element may be operated at lower temperatures. The differential temperature signal may be determined in a known manner. Moreover, these two temperature sensors may be part of a thermal flow sensor and therefore may be further used for measuring a flow of the gas g, if present, for determining the flows as described below.

In some preferred embodiments, said first and second temperature sensors and said heater element are arranged on a bridge structure or on a membrane spanning a cavity in a substrate of said thermal sensor device. The first and second temperature sensors are preferably arranged such that, during measurement, they are spaced in a direction of gravitation. This measurement configuration could be called a vertical configuration. The heater may be arranged in or offset of a middle position of a distance between the two temperature sensors. Alternatively, a horizontal configuration may be used, *i.e.* the temperature sensors are spaced in a direction at right angles with gravitation. In a horizontal configuration, either the geometry of the device may be chosen such that the natural convection flows establish a temperature pattern that allows for determination of a significant signal that allows for determination of the natural-convection parameter $\frac{1}{vD}$. Preferably, the heater element is placed offset of said middle point, *e.g.* by 5% to 30% of a distance between the first and second temperature sensors. The asymmetric placement of the heater between the temperature sensors ensures that the two temperature sensors read temperatures, which's difference is correlated to the natural-convection parameter $\frac{1}{vD}$, wherein said correlation may be determined by means of calibration measurements (e.g. by using the expansion calculation as taught herein).

The method further comprises:

determining a first temperature value $T_1$ at said first temperature sensor and a second temperature value at the second temperature sensor under action of the heater element;
calculating said heat-transfer parameter, e.g. said thermal conductivity λ of said gas, said heat-capacity related parameter, *e.g.* said thermal diffusivity *D* or volumetric specific heat $c_pρ$, said natural-convection parameter $\frac{1}{vD}$, and/or said gas parameter taking into account said first and second temperature values $T_1$, $T_2$.

In some embodiments, said heat-transfer parameter is the thermal conductivity λ and/or said heat-capacity related parameter is the thermal diffusivity D or a volumetric heat capacity $c_pρ$ of said gas of interest.

In a further embodiment of the present invention, furthermore, an absolute pressure of the gas is measured and a combustion parameter is determined.

Accordingly, in some embodiments, additionally, an absolute pressure p of said gas is used or determined. The absolute pressure may be an input that is used or it may be a parameter that is determined by a pressure sensor or a pressure meter that is part of the device. A combustion parameter of said gas may then be calculated from said heat-transfer parameter, said heat-capacity related parameter, said natural-convection parameter $\frac{1}{vD}$, and said absolute pressure p of said gas. This may be done as taught, for example, in WO 2015/075278 A1 or in EP 3 153 854 A1.

Accordingly, the regression parameters are determined, for example, by means of a table of natural gases and gas parameters of these gases. Since, generally, these parameters depend on temperature *T* and pressure *p*, *T* and *p* are measured and considered. The temperature *T* may be measured on the chip, *e.g.* by a temperature sensor in the ASIC part; the pressure may be evaluated from a commercial pressure sensor or set by a pressure meter.

In some embodiments, said combustion parameter is selected from the group comprising:

a calorific value, preferably, heat of combustion per unit volume *Hp;*
a Wobbe index $I_W$; and
a methane number $N_M$.

In some embodiments, said gas parameter is the Prandtl number *Pr* of said gas. For at least some natural gases, the hydrogen concentration may be directly inferred from the Prandtl number *Pr*. Accordingly, the present invention provides a simply and cost-efficient sensor for determining a hydrogen concentration of natural gas.

In a further embodiment of the present invention, a volumetric flow or velocity of said gas is measured by means of a flow sensor. The flow sensor may be an extra flow sensor. Preferably, however, the aforementioned first and second temperature sensors and the heater element therebetween are used, in a known manner, to determine the flow. In some embodiments, from said volumetric flow a volumetric flow at standard temperatures is determined. Accordingly, by including said flow sensor to the thermal sensor device, different flows including, for example, a volumetric flow, a volumetric flow at standard temperatures, a standard volumetric flow, or an energy flow may be determined.

Accordingly, in some embodiments, said thermal sensor device is further configured as a thermal flow sensor. The thermal flow sensor may be configured in a manner as disclosed in WO 01/98736 A1, EP 2 527 779 A1 or WO 2012/021999 A1. Said method then further comprises:

(i) establishing a gas flow in said gas;
(ii) operating said thermal flow sensor exposed to said gas flow and generating a flow signal;
(iii) establishing a zero flow in said gas;
(iv) operating said thermal flow sensor (i.e. activate the heater element to create the natural convection (i.e. the self-convection) and read out the temperature sensors) exposed to said zero flow and generating a zero flow signal; wherein:
(v) the thermal conductivity $\lambda$ and the thermal diffusivity D of said gas are calculated from said output of the thermal sensor device;

(vi) said natural-convection parameter $\dfrac{1}{\nu D}$ is calculated based on said zero flow signal;

(vii) the Prandtl number *Pr* is calculated from said natural-convection parameter $\dfrac{1}{\nu D}$ and said thermal diffusivity (D) of said gas; and wherein:
a volumetric flow or a flow velocity is calculated based on said flow signal of the flow sensor, said thermal conductivity $\lambda$, said thermal diffusivity *D*, and said Prandtl number *Pr* of said gas.

Alternatively or additionally, a volumetric flow at standard temperature may be calculated based on said temperature, said thermal conductivity $\lambda$, said thermal diffusivity *D*, and said Prandtl number *Pr* of said gas.

The thermal flow sensor may be a standard thermal flow sensor including two temperature sensors and a heater element therebetween, whilst these components are arranged on a bridge or on a membrane structure. A membrane-type design and a bridgetype design of a thermal flow sensor are described in more detail in WO 01/98736 A1, US 2004/0099057 A1, EP 1 840 535, and in US 5,050,429 A, respectively.

In a further embodiment, the thermal sensor is or includes a thermal flow sensor and an absolute pressure of the gas is used or determined, e.g. by an additional pressure sensor, whereupon a flow related parameter is determined considering the absolute pressure of the gas.

Accordingly, in some embodiments, additionally, the absolute pressure p of said gas is used or determined; wherein a standard volumetric flow *Q* is calculated based said temperature of the gas, said absolute pressure *p*, and said volumetric flow *V*.

In some embodiments, the absolute pressure *p* of said gas is used or determined; wherein an energy flow $J_E$ is calculated based on said combustion parameter as mentioned above and said volumetric flow *V*.

In a further aspect, the present invention relates to a thermal sensor device that is able to determine a gas parameter based on calculation inputs including measured values such as the natural-convection parameter. More specifically, the present invention relates to a thermal sensor device for determining a gas parameter of a gas, the thermal sensor device comprising:

at least one temperature sensor and at least one heater element and being exposed to said gas, said at least one temperature sensor and said at least one heater element being preferably arranged on a bridge structure or on a membrane spanning a cavity in a substrate of said thermal sensor device;
a control device configured to carry out the following method:

- operating said thermal sensor device being exposed to said gas;

- calculating from an output of said thermal sensor device:

(i) a temperature of said gas;

(ii) a heat-transfer parameter of said gas, wherein said heat-transfer parameter of said gas is determined from a stationary temperature distribution in said gas caused by said at least one heater element;

(iii) a heat-capacity related parameter of said gas, wherein said related parameter of said gas is determined from a time-varying heating scheme applied to said heater element;

(iv) a natural-convection parameter $\frac{1}{\nu D}$ of said gas related to a natural convection caused in said gas by action of said at least one heater element; and

- calculating said gas parameter of said gas from said heat-transfer parameter, preferably the thermal conductivity $\lambda$ of the gas, said heat-capacity related parameter, preferably a thermal diffusivity $D$ or a volumetric specific heat $c_p \rho$, and said natural-convection parameter $\frac{1}{\nu D}$ .

In some embodiments, said control device of the thermal sensor device may be configured to carry out the method according to any of the herein described embodiments of the present invention.

In some embodiments, the thermal sensor device comprises two or more temperature sensors, wherein one or more of said at least one heater element is arranged between said temperature sensors and off-set from a middle point between said temperature sensors, preferably by 5% to 30% of a distance between said temperature sensors. The offset arrangement causes a different temperature signal at the first and at the second temperature sensor, wherein the difference may be used as a measure of the natural-convection parameter related to convection flows established in the gas of interest due to the action of the heater element.

In some embodiments, the thermal sensor device is configured such that, when in use is use, said temperature sensors are arranged at a distance along the direction of gravitation. This arrangement allows for temperature readings from the first and second temperatures sensors for calculating the natural-convection related parameter. In such a configuration, the heater element may be arranged in the middle of the distance between the two temperature sensors; alternatively, it may be arranged offset of this middle point, *e.g.* by 5% to 30% of said distance between said temperature sensors.

In a further embodiment, the present invention relates to a thermal sensor system comprising the thermal sensor device according to invention and further comprising an absolute pressure sensor or a pressure meter for measuring and controlling, respectively, an absolute pressure p of said gas. The control device is further configured to carry out the method according to invention, for example for determination of a combustion parameter, the Prandtl number, or the hydrogen concentration of the gas as explained above.

In some embodiments, the thermal sensor system further comprises a thermal flow sensor device, wherein said control device is configured to carry out the method according to invention for determining a volumetric flow, a flow velocity, a volumetric flow at standard temperature, a standard volumetric flow, and/or an energy flow. Accordingly, in some embodiments, additionally, the thermal sensor system is configured for using or determining the absolute pressure $p$ of said gas, wherein a standard volumetric flow $Q$ is calculated based said temperature of the gas, said absolute pressure p, and said volumetric flow $V$. In some embodiments, additionally, the thermal sensor system is configured for using or determining the absolute pressure $p$ of said gas, wherein an energy flow $J_E$ is calculated based on said combustion parameter as mentioned above and said volumetric flow $V$.

In a further aspect, the present invention relates to a non-transitory computer readable medium for thermal sensor device according to invention, said non-transitory computer readable medium being encoded with a computer program product comprising computer code that, when executed in a processor, carries out the method according to invention.

The computer code may be provided in a source code, in a machine-executable code, or in any intermediate form of code-like object code. It can be provided as a computer program product on a computer-readable medium in tangible form, *e.g.* on a CD-ROM or on a Flash ROM memory element or similar, or it can be made available in the form of a network-accessible medium for download from one or more remote servers through a network.

It is to be understood that these aspects will be better understood when considered with the description of the preferred embodiments below. Aspects may be combined with one another without departing from the scope of the appended claims and further embodiments may be formed with parts or all the features of the embodiments as described herein.

BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

Fig. 1     shows a schematic functional diagram of a thermal sensor device according to an embodiment of the present invention;

Fig. 2     shows in a schematic perspective view a thermal flow sensor according to a first embodiment that may be employed in the present invention;

Fig. 3     shows in a schematic perspective view a thermal flow sensor according to a second embodiment that may be employed in the present invention;

Fig. 4     shows in a schematic perspective view a thermal flow sensor according to a third embodiment that may be employed in the present invention;

Fig. 5     shows a flow diagram of an embodiment of the method according to the present invention;

Fig. 6     correlation between the correlation calculation and $Hp$;

Fig. 7     the relative error related to Fig. 6;

Fig. 8     the relative error when calculating the volume percentage of $H_2$ gas in $CH_4$ gas;

Fig. 9     shows a diagram that correlates the volume percentage of $H_2$ gas in $CH_4$ gas and the corresponding Prandtl number; and

Fig. 10    shows a simplified block diagram of a digital control circuit that can be employed in the present invention.

## DESCRIPTION OF PREFERRED EMBODIMENTS

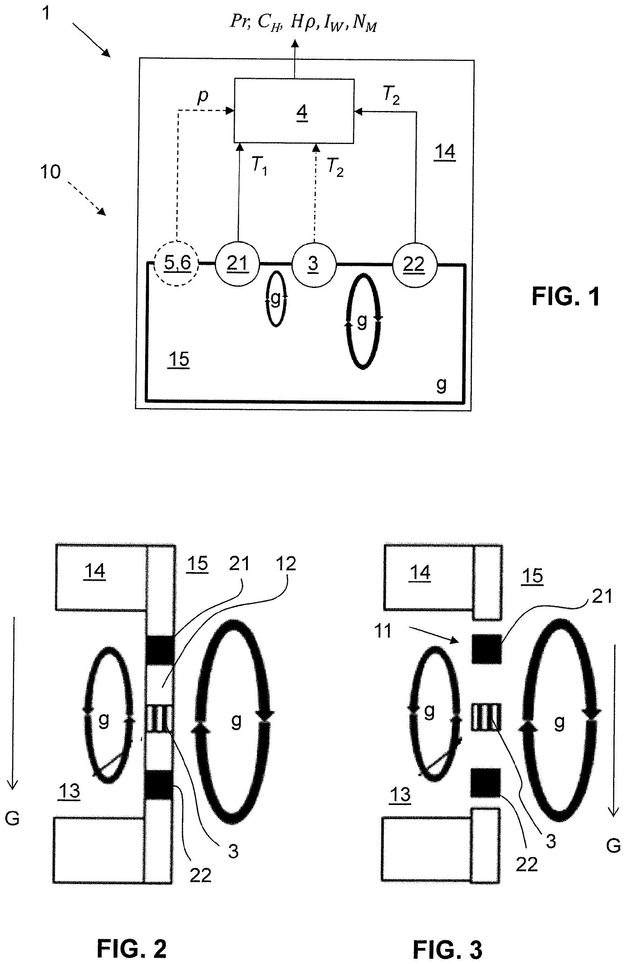

**Figure 1** shows a schematic functional diagram of a thermal sensor device 1 according to an embodiment of the present invention. A gas g of interest is present in volume 15 and is exposed to a heater element 3. Natural convection due to heat and gravitation establishes (no other significant flow driver is present). First and second temperature sensors 21, 22 are arranged, one on either side of the heater element 3 (*e.g.* a resistive path or wire), for example, on a bridging structure 11 or a membrane 12 spanning a cavity in a substrate 14. The first and second temperature sensors 21, 22 may be thermopiles. They pick up the temperatures $T_1$ and $T_2$ of the gas g at respective locations of the sensors 21, 22. Alternatively, one of the temperature sensors 21, 22 is dispensed with and the heater element 3 is used as a further temperature sensor (*e.g.* by reading out its resistance, indicated by the dashed-dotted lines). In some embodiments, see below, an absolute pressure sensor 5 or pressure meter 6 is arranged for determining an absolute pressure p of the gas g (see dashed lines). A control device 4 receives readings from the sensors 21, 22 (in some embodiments 3), and possibly 5 or 6. From these values, the control device 4 calculates a gas parameter of the gas g. According to invention, the following parameters are measured:

(i) a temperature $T$ of said gas g;

(ii) a heat-transfer parameter such as the thermal conductivity $\lambda$ of said gas g;

(iii) a heat-capacity related parameter such as the thermal diffusivity $D$ or the volumetric specific heat $c_p\rho$ of said gas g; and

(iv) a natural-convection parameter $\dfrac{1}{\nu D}$ of said gas g related to a natural convection caused in said gas g by action of said at least one heater element 3.

Then, the Prandtl number $Pr$, an $H_2$ concentration $C_H$, or a combustion parameter such as a heat of combustion per unit volume $Hp$, a Wobbe index $I_w$, or a methane number $N_M$ of said gas g is calculated from said heat-transfer parameter, said heat-capacity related parameter, said temperature T, said natural-convection parameter $\dfrac{1}{\nu D}$, and possibly said absolute pressure p.

Figure 1 is highly schematic. It is to be understood, that both temperature sensors 21, 22 may be arranged on any same side of the heater element 3. The sensors 21, 22 may be arranged on a bridging structure (see Fig. 2) or on a membrane (see Fig. 3) that span a cavity 13. The cavity 13 may be part of the volume 15. The sensors 21, 22 may be part of a thermal flow sensor arrangement as taught, *e.g.*, in EP 1 426 740.

Figures 2, 3 and 4 show preferred embodiments of the thermal sensor device 1.

**Figure 2** shows the thermal sensor device 1 embodied by having a substrate 14 into which a cavity 13 is formed. The cavity 13 is spanned by a membrane 12. On the membrane 12 are arranged the temperature sensors 21, 22 and the heater element 3 for thermally decoupling them from the substrate 14 the latter tending to receive waste heat from electronic component supported thereon. The membrane 12 is arranged vertically, *i.e.* during measurement, the device 1 is oriented such that the membrane 12 extends parallel to the gravitational direction G. The temperature sensors 21, 22 are vertically spaced along the gravitational direction G. In other words: the temperature sensors 21, 22 are arranged on different heights (with respect to the gravitational direction G). The heater element 3 is arranged in-between the two temperature sensors 21, 22.

**Figure 3** shows a second embodiment, where instead of a vertical membrane 12 a bridging structure 11 is spanning the cavity 13, wherein the first and second temperature sensors 21, 22 and the heater element 3 are arranged on the bridging structure 11 for decoupling it from the substrate 14.

In the Figs. 2 and 3, the heater element 3 may be arranged at or offset (by 5% to 30% or a distance or 21 to 22) to a middle point P between the first and second temperature sensors 21, 22.

**Figure 4** shows a third embodiment, where the bridging structure 11 is extending at right angles to the gravitational direction G, at least during measurement. Accordingly, the first and second temperature sensors 21, 22 are horizontally spaced. In order to provide a strong natural-convection signal $S_{con}$, the heater element 3 is arranged offset (by 5% to 30% or a distance or 21 to 22) to a middle point P between the first and second temperature sensors 21, 22. This asymmetric placement causes the first and second temperature sensors 21, 22 to sample different parts of the convection loop established by the heater 3 action in the gas g.

It is also conceivable to use such an asymmetric arrangement of the first and second temperature sensors 21, 22 with respect to the heater element 3 in a vertical arrangement, *i.e.* in devices 1 where the membrane 12 or bridging structure 11 is oriented parallel to the gravitational direction G. This would further enhance the precision of the measurement.

**Figure 5** shows possible implementations of the method according to invention.

In step 601, the temperature T may be determined by using the first or second temperature sensor 21, 22 of the thermal sensor device 1 according to any one of Figs. 2 to 4 or another temperature sensor that may be arranged on the substrate of the sensor chip with the thermal sensor device 1, for example, on its ASIC part.

In step 602, the heater element 3 is operated and the thermal conductivity $\lambda$ of the gas g is calculated from signal related to a temperature/heater measurement scheme as taught, for example, in US 6,019,505. Accordingly, the maximum temperature measured at the first/and or second temperature sensors 21, 22 (or at sum or difference of the maximum temperatures measured at the first/and or second temperature sensors 21, 22) with the heater element 3 at a predefined setting (e.g. at maximum heating power) is an inverse function of the thermal conductivity $\lambda$. The inverse function may be determined by means of calibration measurements. In step 603, the heater element 3 is operated and the thermal diffusivity *D* of the gas g which is from signal related to a temperature/heater measurement scheme as taught, for example, in US 6,019,505. Accordingly, the first and second temperature sensors 21, 22 are spaced at different distances to the heater element 3 and measure the propagation of a heat wave generated by means of the heater element 3. The phase difference between the generated heat wave at the heater element 3 and the first and/or second temperature sensor 21/22 or the phase difference of said wave between the two temperature sensors (as shown *e.g.* in Fig. 4) is a function of the thermal diffusivity *D*. This function may be determined by means of calibration measurements.

In step 604, the natural-convection parameter $\frac{1}{\nu D}$ is determined by establishing a zero-flow $f_0$ situation and turning on said at least one heater element 3, preferably to heat at constant power, whereby the macroscopic flow in the gas g is due to the action of the heater element 3, said macroscopic flow being a natural convection flow as indicated by the elliptical closed arrows in the Figs. 1 to 4. From the temperature readings $T_1$, $T_2$ of the first and second temperature sensors 21, 22, a temperature difference (or sum) is calculated and used as natural-convection signal $S_{con}$. This natural-convection signal $S_{con}$ is then used by the control device 4 for calculating the natural-convection parameter $\frac{1}{\nu D}$. This calculation is based on polynomial expansion. Specifically, the natural-convection parameter related signal $S_{con}$ may be written in an expansion of the natural-convection parameter $\frac{1}{\nu D} = \frac{c_p \rho^2}{\lambda \eta}$ as follows:

$$S_{con} = a + b \left( \frac{1}{\nu D} \right) + c \left( \frac{1}{\nu D} \right)^2 + \cdots$$

wherein expansion coefficients *a, b, c* etc. are functions of the thermal conductivity $\lambda$ and the temperature *T* of the gas. The coefficients *a, b,* c etc. were determined beforehand and made available for calculation by means of calibration measurement and stored in one or more lookup tables LUT .

In step 605, the thermal conductivity $\lambda$ and the gas temperature T, as determined by the control device 4 in steps 602 and 601, respectively, and the expansion coefficients a, *b,* c etc. as retrieved from one or more lookup tables LUT are then used for calculating from the natural-convection related signal $S_{con}$ of the thermal sensor device 1 said natural-convection parameter $\frac{1}{vD}$ by using one of the following formula derived by linear expansion or a quadratic expansion

- linear expansion: $\frac{1}{vD} = \frac{(S_{con}-a)}{b}$ ; and

- quadratic expansion: $\frac{1}{vD} = \frac{1}{2c}\left(-b + \sqrt{b^2 - 4c(a - S_{con})}\right)$ , respectively.

In step 606, the gas parameter is calculated. From the natural-convection parameter $\frac{1}{vD}$ and the thermal diffusivity D one readily obtains, for example, the Prandtl number $Pr = \frac{v}{D} = \frac{1}{D^2\left(\frac{1}{vD}\right)}$ .

In step 607, the gas parameter is output to the user or further devices.

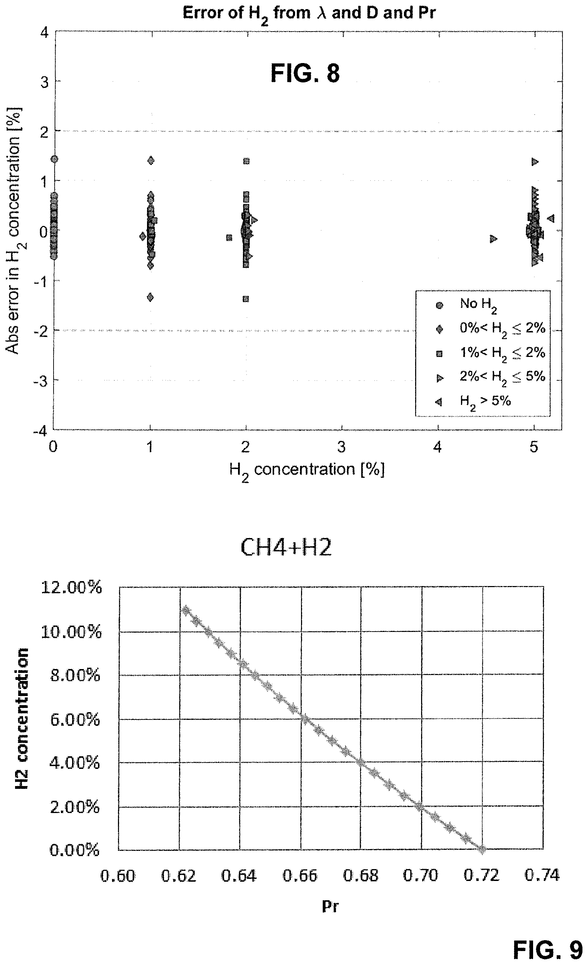

In some embodiments, from the Prandtl number *Pr* one may determine, *e.g.* by means of a lookup table that stores a beforehand established correlation of the Prandtl number and the hydrogen concentration, a hydrogen concentration $C_H$ of a natural gas g of interest. The hydrogen concentration is the volume percentage of $H_2$ gas in the natural gas. Such a correlation is depicted in **Figure 9.** Accordingly, this thermal sensor device 1 may be used to identify a gas that is, for example, a binary mixture of a natural gas and a $H_2$ gas.

In a further development, a thermal sensor system 10 (see Fig. 1, dashed lines) may comprise such a thermal sensor device 1 and may be further configured that the absolute pressure p of the gas g is received and considered or is directly measured by means of the absolute pressure sensor 5 or set by means of the pressure meter 6. Acquiring the information on the absolute pressure p is indicated in Fig. 5 as optional step 608 and in Fig. 1 by the dashed lines.

At this point, the control device 4 has available the three parameters (i) thermal conductivity λ, (ii) thermal diffusivity *D,* (iii) Prandtl number *Pr,* and in addition the absolute gas temperature *T* and the absolute gas pressure *p.*

Preferably, the temperature sensor for determining the absolute gas temperature T is arranged on the ASIC part of the sensor chip with the thermal sensor device 1. The absolute pressure may be measured by means of a separate commercially available pressure sensor 5.

The combustion parameter may be determined by using a quadratic Ansatz as taught, for example, in WO 2015/075278 A1, EP 3 153 854 A1 or in EP 2 015 056.

In the following, a specific example shall be demonstrated. The correlation between the parameter set *(λ,D,vD)* and the *Hp,* the heat of combustion per unit volume, shall presented for $CH_4$, $CH_4$ with up to 2 vol.% of $H_2$ gas, $CH_4$ with 2 to 5 vol.% of $H_2$ gas, and $CH_4$ with 5 to 10 vol.% of $H_2$ gas at standard conditions. Under standard conditions - for this example, a standard pressure $p_0$=0.10132 MPa and at a standard temperature T=25°C-, one may us the following regress function:

$$R_{H\rho} = \vec{\beta}_{H\rho} \cdot \vec{x},$$

with $\vec{x} = (1, x_1, x_1^2, x_2, x_2^2, x_3, x_3^2, x_1 x_2, x_1 x_3, x_2 x_3)^T$ , where $x_1 = λ/λ_{CH4}$, $x_2 = D/D_{CH4}$ and $x_3 = vD/(vD)_{CH4}$. $CH_4$ denotes methane.

Through minimization of the root-mean-square error one finds:

$$\vec{\beta}_{H\rho} = (30.7, 596.5, -683.9, -901.4, 68.8, 336.4, 165.4, 1023, -104.3, -495.1)^T \frac{MJ}{Sm^3}.$$

The unit here is megajoule per standard cubic meter, the standard cubic meter being the volume of 1 cubic meter of the gas g at standard conditions.

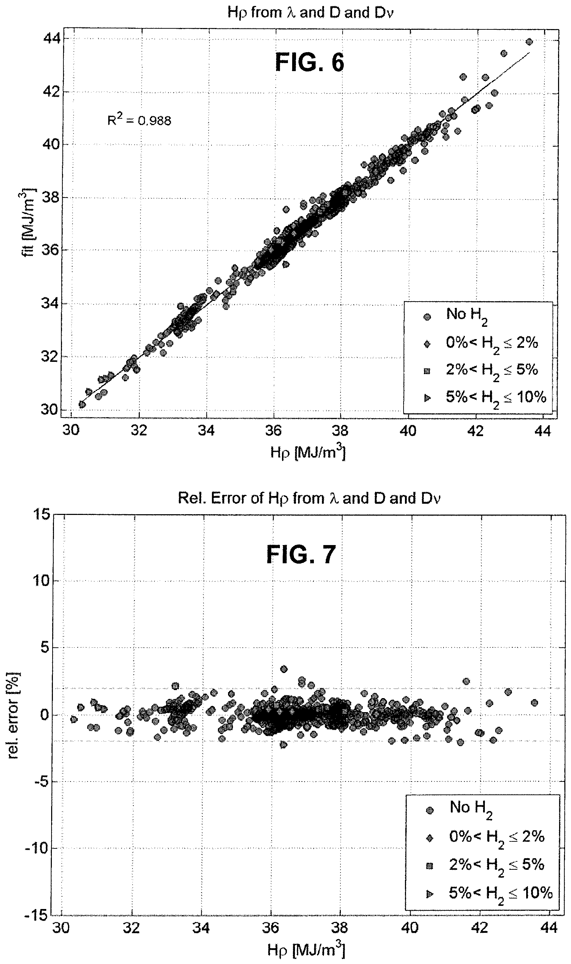

**Figure 6** shows the correlation between the fit and *Hp,* **Figure 7** the relative error. The correlation is obvious, the correlation coefficient being $R^2 = 0.988$.

$\vec{\beta}_{H\rho}$ depends on absolute gas temperature *T* and the absolute gas pressure *p.*

$\vec{\beta}_{Hp}(T,p)$ may be determined by means of calibration and stored in the control device 4 in one or more lookup tables LUT.

Other combustion parameters such as the Wobbe index $I_w$, the methane number $N_M$, or the like may be determined as taught, for example, in WO 2015/075278 A1, EP 3 153 854 A1 or in EP 2 015 056.

Moreover, the $H_2$ concentration $C_H$ in a natural gas such as methane may be determined in a correlative manner. Using again the formula:

$$R_{H_2} = \vec{\beta}_{H_2} \cdot \vec{x}$$

with $\vec{x} = (1, x_1, x_1^2, x_2, x_2^2, x_3, x_3^2, x_1 x_2, x_1 x_3, x_2 x_3)^T$ , where $x_1 = \lambda/\lambda_{CH4}$, $x_2 = D/D_{CH4}$ and $x_3 = Pr/Pr_{CH4}$ one finds through minimization of the root-mean-square error:

$$\vec{\beta}_{H_2} = (-3.76, 3.29, 0.63, -0.13, 0.98, 4.90, -1.67, -2.08, -2.40, 0.25)^T$$

**Figure 8** shows the relative error of this correlation.

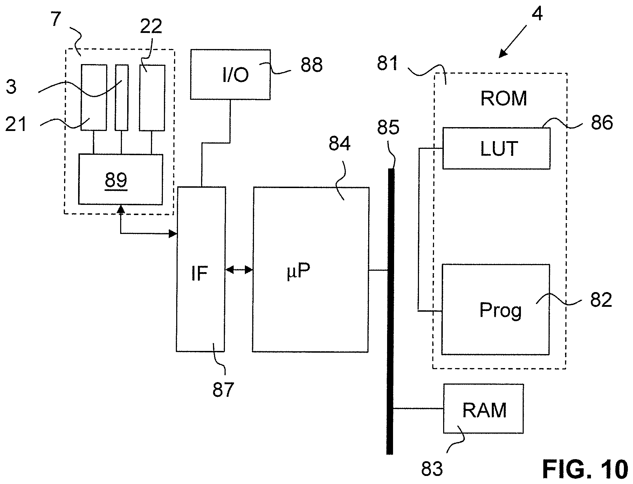

From this coefficient set $\vec{\beta}_{H2}$ one can see that $x_3 = 4.9$ has a dominating weight, which is why the Prandtl number being associated with $x_3$ has a dominating weight and may therefore be used as direct measurement for the $H_2$ concentration $C_H$ as shown in Figure 10, showing a binary gas mixture of $CH_4$ and $H_2$. A physical explanation would be that the $H_2$ molecules are considerably smaller than the $CH_4$ molecules.

In a further development, the thermal sensor system 10 may comprise a flow sensor 7 for determining a volumetric flow $\dot{V} = \dfrac{dV}{dt}$ of the gas g. It may comprise the pressure sensor 5 or meter 5 but does not have to.

The zero-flow condition for the measurement of the natural convection parameter or other parameters may be established in the flow channel or it may be established in a dead volume in communication with the flow channel during the flow of the gas through the flow channel.

If then a gas flow of the gas g is present, the flow sensor 7 picks up a flow signal $S_{flow}$. This flow signal $S_{flow}$ is related to the volumetric flow $V$ by the following relation:

$$S_{flow} = F_{\lambda,Pr}\left(\frac{\dot{V}}{D}\right).$$

with $F_{\lambda,Pr}$ being a function depending on the thermal conductivity $A$ and the Prandtl number $Pr$ that can be reversed, wherein the volumetric flow $V$ may be written by using the reversed function $F_{\lambda,Pr}^{-1}$ as determined by means of calibration measurements and available through, for example, a lookup table, and the thermal diffusivity D of the gas g as:

$$\dot{V} = D\, F_{\lambda,Pr}^{-1}\left(S_{flow}\right).$$

If the gas of interest is known, the function $F_{\lambda,Pr}$ and/or its reverse function $F_{\lambda,Pr}^{-1}$ may be determined by calibration measurements and made available to the system 10 by storing it to the non-volatile memory. One may use a lookup table. If the gas is unknown, a parameter set $D, \lambda, Pr$ may first be determined of the unknown gas g. The reverse function $F_{\lambda,Pr}^{-1}$ may then be determined, for example, by applying a two-dimensional linear interpolation in the $(\lambda, Pr)$ space. In practice, one may use, e.g., three different lookup tables for three different gases which span a triangle in the $(\lambda, Pr)$ space. The flow signal $S_{flow}$ is then linearized in each of the three lookup tables and the final flow value is obtained as the convex combination. This procedure is analogous to the ID interpolation detailed in EP 3 153 854 A1 (Eq. 6). The volumetric flow V may then be calculated by the control device 4 and may be output in step 607.

From the volumetric flow $V$ a volumetric flow at standard temperature, $F$, may be calculated, the standard temperature $T_0$ being, for example, $T_0=25°C$, by the control device 4, by using the relation:

$$F = \frac{T_0}{T}\dot{V}$$

If, furthermore, the absolute pressure p of the gas is known (by receiving or measuring it), the following flows may be calculated by the system 10:

Standard volumetric flow: $Q = \frac{T_0}{T}\frac{p}{p_0}\dot{V}$, and

Energy flow: $J_E = (H\rho) * \dot{V} = (H\rho_0) * Q = \left(\frac{\lambda H}{c_p}\right) * \left(\frac{\dot{V}}{D}\right)$ $H\rho$ may be determined as taught above.

These flows may be outputted to a user or used by further devices as the gas parameter of interest.

**Figure 10** shows a schematic block diagram of a digital control device 4. The control device 4 comprises a processor (CPU, $\mu$P) 84, a non-volatile (e.g. Flash ROM) memory 81, and a volatile (RAM) memory 83. The processor 84 communicates with the memory modules 81, 83 via one more data buses 85. The non-volatile memory 81 stores, inter alia, sets of calibration data 86, e.g. in form of one or more lookup tables LUT, and other data required for the calculation of the target gas parameter, as well as a machine-executable program 82 for execution in the processor 84. The calibration data may include a correlation between a hydrogen concentration $C_H$ (i.e. volume % of $H_2$ gas in the gas g) and a Prandtl number $Pr$ of one or more gases. It may contain expansion coefficients (in dependence of temperature T and thermal conductivity $\lambda$ of the gas) for calculation of the natural-convection related parameter $\frac{1}{\nu D}$. It may contain a reverse function for determining a volumetric flow $V$, a volumetric flow F at standard temperatures, a standard volumetric flow $Q$, or an energy flow $J_E$.

The processor 84 may communicate, through a device interface (IF) 87, with various peripherals, which may include, for example, at least one input/output (I/O) interface 88 for interfacing with an external input/output device such as a keyboard and/or mouse, an LCD screen, etc., with the first and second temperature sensors 21, 22, with the heater element 3, and, if any, with the pressure sensor 5, the pressure meter 6, or the flow sensor 7, via its built-in digital circuitry 89.

Accordingly, the present invention relates to a device that comprises a thermal sensor 1, the thermal sensor being preferably a thermal flow sensor, with two or more temperature sensors 21, 22 and a heater element 3 arranged therebetween, the temperature sensors 21, 22 and the heater element 3 may be arranged on a membrane 12 or on a bridging structure 11. By means of the thermal sensor, the parameter set *(D, $\lambda$, Pr)* (or an equivalent set of parameters such as, for example, *(D, $\lambda$, $c_p\rho$))* may be determined as described herein. The temperature *T* of the gas g may be determined by means of the first or second temperature sensor 21, 22 or by use of a further temperature sensor that may be arranged, for example, in the ASIC part of the chip that carries the thermal flow sensor. Moreover, in some embodiment, the volumetric flow or flow velocity may be determined by means of the flow sensor and various flows as described herein may be measured with the device 1 or system 10. Further, in a preferred embodiment, an absolute pressure sensor 5 or pressure meter 6 is added to the device 1, thereby creating a simple thermal sensor system 10. By means of the absolute pressure sensor value *p,* combustion parameters such as the Wobbe index, the methane number, or the combustion heat or others become available as gas parameter output of the system 10. The flow sensor and pressure sensor may be included separately to the thermal sensor 1 or both together, depending on the required output gas parameter. The control device 4 is configured to perform the calculations as outlined above for the different embodiments of the device 1 or system 10. The device 1 or system 10 is small, compact, cost-efficient and reliable. By means of the system 10, a gas quality system may be provided. In some embodiments, this device 1 or system 10 allows for determination of an $H_2$ concentration in a gas.

LIST OF REFERENCE SIGNS

| 1 | thermal sensor device | 88 | input/output interface |
|---|---|---|---|
| 10 | thermal sensor system | 89 | circuitry |
| 11 | bridge structure | $C_H$ | Hydrogen concentration in g |
| 12 | membrane | $c_p\rho$ | volumetric heat capacity |
| 13 | cavity in 14 | d | distance between 21, 22 |
| 14 | substrate of 1 | D | thermal diffusivity |
| 15 | volume | f | gas flow |
| | | $f_0$ | zero gas flow |
| 2 | temperature sensor | g | gas |

(continued)

| | | | |
|---|---|---|---|
| 21 | first temperature sensor | $H\rho$ | Calorific value of g |
| 22 | second temperature sensor | $I_w$ | Wobbe index of g |
| | | $J_E$ | energy flow |
| 3 | heater element | $N_M$ | Methane number of g |
| 4 | control device | p | absolute pressure of g |
| 5 | pressure sensor | P | middle point of d |
| 6 | pressure meter or controller | Pr | Prandtl number of g |
| 7 | thermal flow sensor | Q | standard volumetric flow |
| | | $S_{con}$ | natural-convection signal |
| 601-609 | method steps | $S\backslash_{flow}$ | flow signal |
| | | $S_{flow,0}$ | zero flow signal |
| 81 | non-transitory computer readable medium, non-volatile memory | T | temperature value of g |
| | | $T_1$ | first temperature value |
| | | $T_2$ | second temperature value |
| 82 | computer program product | $\dot{V}$ | volumetric flow |
| 83 | volatile memory | v | flow velocity |
| 84 | processor $\frac{1}{vD}$ | | natural-convection parameter |
| 85 | bus | $\lambda$ | thermal conductivity of g |
| 86 | calibration data, lookup table(s) LUT | | |
| 87 | device interface | | |

**Claims**

1. A method for determining a gas parameter $(Pr;C_H;H\rho; I_W; N_M)$ of a gas (g), wherein said gas parameter $(Pr;C_H;H\rho; I_W; N_M)$ is a Prandtl number, a hydrogen concentration, or a combustion parameter of the gas (g), said method comprising:

   - operating a thermal sensor device (1) being exposed to said gas (g) and comprising at least one temperature sensor (2;21,22) and at least one heater element (3);
   - calculating from an output of said thermal sensor device (1):

     (i) a temperature $(T)$ of said gas (g);
     (ii) a heat-transfer parameter $(\lambda)$ of said gas (g), wherein said heat-transfer parameter $(\lambda)$ of said gas (g) is determined from a stationary temperature distribution in said gas (g) caused by said at least one heater element (3);
     (iii) a heat-capacity related parameter $(D; c_p\rho)$ of said gas (g), wherein said heat-capacity related parameter $(D; c_p\rho)$ of said gas (g) is determined from a time-varying heating scheme applied to said heater element (3);
     **characterized in that** the method further comprises calculating from the output of said thermal sensor device (1):

     (iv) a natural-convection parameter $\left(\frac{1}{vD}\right)$ of said gas (g) related to a natural convection caused in said gas (g) by action of said at least one heater element (3); and

   - calculating said gas parameter $(Pr;C_H;H\rho; I_W; N_M)$ of said gas (g) from said heat-transfer parameter $(\lambda)$, said heat-capacity related parameter $(D; c_p\rho)$, and said natural-convection parameter $\left(\frac{1}{vD}\right)$.

2. The method according to claim 1, wherein said natural-convection parameter $\left(\frac{1}{vD}\right)$ is calculated from said heat-transfer parameter $(\lambda)$ and said temperature $(T)$ of said gas (g), preferably by using an expansion of an output $(S_{con})$

of said thermal sensor device (1) related to the natural-convection parameter $\left(\frac{1}{\nu D}\right)$ in said natural-convection parameter $\left(\frac{1}{\nu D}\right)$ ;

wherein, preferably, expansion terms of said expansion are empirically determined functions of said heat-transfer parameter ($\lambda$) and said temperature *(T)* of said gas (g); and/or
wherein, preferably, said expansion is made up to a linear term or to a quadratic terms.

**3.** The method according to any one of claims 1 to 2, wherein said thermal sensor device (1) comprises, as said at least one heater element (3), a first temperature sensor (21) and a second temperature sensor (22), said heater element (3) being arranged therebetween, said first and second temperature sensors (21,22) and said heater element (3) being preferably arranged on a bridge structure (11) or a membrane (12) spanning a cavity (13) in a substrate (14) of said thermal sensor device (1);
and wherein said method comprises:

determining a first temperature value $(T_1)$ at said first temperature sensor (21) and a second temperature value $(T_2)$ at the second temperature sensor (22) under action of the heater element (3);
calculating said heat-transfer parameter ($\lambda$), said heat-capacity related parameter $(D; c_p\rho)$, said natural-convection parameter $\left(\frac{1}{\nu D}\right)$ , and/or said gas parameter $(Pr; C_H; H\rho; I_W; N_M)$ taking into account said first and second temperature values $(T_1, T_2)$.

**4.** The method according to claim 3, wherein the heater element (3) is arranged between said first and second temperature sensors (21,22) in a manner off-set from a middle point (P) between said temperature sensors (21,22), preferably by 5% to 30% of a distance (d) between said temperature sensors (21,22); and/or
wherein the thermal sensor device (1) is used such that said first and second temperature sensors (21,22) are separated along the direction of gravitation.

**5.** The method according to any one of claims 1 to 4, wherein said heat-transfer parameter ($\lambda$) is a thermal conductivity ($\lambda$) and/or said heat-capacity related parameter $(D; c_p\rho)$ is a thermal diffusivity $(D)$ or a volumetric heat capacity $(c_p\rho)$ of said gas (g).

**6.** The method according to any one of claims 1 to 5, wherein, additionally, an absolute pressure *(p)* of said gas (g) is used or determined, and as said gas parameter a combustion parameter $(Hp; I_W; N_M)$ of said gas (g) is calculated from said heat-transfer parameter $(\lambda)$, said heat-capacity related parameter $(D; c_p\rho)$, said natural-convection parameter $\left(\frac{1}{\nu D}\right)$ , and said absolute pressure *(p)* of said gas (g).

**7.** The method according to claim 6, wherein said combustion parameter $(H\rho; I_W; N_M)$ is selected from the group comprising:

a calorific value, preferably, a heat of combustion per unit volume *(Hp)*;
a Wobbe index $(I_W)$; and
a methane number $(N_M)$.

**8.** The method according to any one of the claims 1 to 7, wherein said gas parameter is the Prandtl number (Pr) of said gas (g), or wherein said gas (g) is a natural gas and said gas parameter is a hydrogen concentration $(C_H)$ of said gas (g).

**9.** The method according to claim 8, said thermal sensor device (1) being further configured as a thermal flow sensor (7), wherein said method further comprises:

(i) establishing a gas flow (*f*) in said gas (g);
(ii) operating said thermal flow sensor (7) exposed to said gas flow (f) and generating a flow signal $(S_{flow})$;
(iii) establishing a zero gas flow $(f_0)$ in said gas (g), preferably at constant heating power to the heating element (3);

(iv) operating said thermal flow sensor (7) exposed to said zero gas flow ($f_0$) and generating a zero flow signal ($S_{flow,0}$);

wherein:

(v) the thermal conductivity ($\lambda$) and the thermal diffusivity (D) of said gas (g) are calculated from said output of the thermal sensor device (1);

(vi) said natural-convection parameter $\left(\frac{1}{\nu D}\right)$ is calculated based on said zero flow signal ($S_{flow,0}$);

(vii) the Prandtl number (Pr) is calculated from said natural-convection parameter $\left(\frac{1}{\nu D}\right)$ and said thermal diffusivity (D) of said gas (g);

and wherein:

(vii) a volumetric flow ($\dot{V}$) or a flow velocity (v) is calculated based on said flow signal ($S_{flow}$), said thermal conductivity ($\lambda$), said thermal diffusivity (D), and said Prandtl number (Pr) of said gas (g); and/or
(viii) a volumetric flow at standard temperature (F) is calculated based on said flow signal ($S_{flow}$), said temperature ($\lambda$), said thermal conductivity ($\lambda$), said thermal diffusivity (D), and said Prandtl number (Pr) of said gas (g).

10. The method according to claim 9, wherein, additionally, an absolute pressure (p) of said gas (g) is used or determined by means of an absolute pressure sensor (5) or a pressure meter (6); wherein:

(i) a standard volumetric flow (Q) is calculated based said temperature (T), said absolute pressure (p), and said volumetric flow (V); and/or
(ii) an energy flow ($J_E$) is calculated based on said combustion parameter ($Hp$; $I_W$; $N_m$) according to claim 8 or 9 and said volumetric flow (V).

11. A thermal sensor device (1) for determining a gas parameter ($Pr$;$C_H$;$Hp$; $I_W$; $N_M$) of a gas (g), wherein said gas parameter ($Pr$;$C_H$;$Hp$; $I_W$; $N_M$) is a Prandtl number, a hydrogen concentration, or a combustion parameter of the gas (g), the thermal sensor device (1) comprising:

at least one temperature sensor (2;21,22) and at least one heater element (3) and being exposed to said gas (g), said at least one temperature sensor (2;21,22) and said at least one heater element (3) being preferably arranged on a bridge structure (11) or on a membrane (12) spanning a cavity (13) in a substrate (14) of said thermal sensor device (1);
a control device (4) configured to carry out the following method:

- operating said thermal sensor device (1) being exposed to said gas (g);
- calculating from an output of said thermal sensor device (1):

(i) a temperature (T) of said gas (g);
(ii) a heat-transfer parameter ($\lambda$) of said gas (g), wherein said heat-transfer parameter ($\lambda$) of said gas (g) is determined from a stationary temperature distribution in said gas (g) caused by said at least one heater element (3);
(iii) a heat-capacity related parameter (D; $c_p\rho$) of said gas (g), wherein said heat-capacity related parameter (D; $c_p\rho$) of said gas (g) is determined from a time-varying heating scheme applied to said heater element (3);
**characterized in that** the control device (4) is further configured to calculate from the output of said thermal sensor device (1):

(iv) a natural-convection parameter $\left(\frac{1}{\nu D}\right)$ of said gas (g) related to a natural convection caused in said gas (g) by action of said at least one heater element (3); and

- calculating said gas parameter ($Pr$;$C_H$;$Hp$; $I_W$; $N_M$) of said gas (g) from said heat-transfer parameter ($\lambda$),

said heat-capacity related parameter (D; $c_p\rho$), and said natural-convection parameter $\left(\frac{1}{\nu D}\right)$;

wherein, preferably, said control device (4) is further configured to carry out the method according to any one of the claims 2 to 5 or 8.

12. The thermal sensor device (1) according to claim 11, comprising two or more temperature sensors (21,22), wherein one or more of said at least one heater element (3) is arranged between said temperature sensors (21,22) and off-set from a middle point (P) between said temperature sensors (21,22), preferably by 5% to 30% of a distance (d) between said temperature sensors (21,22); and/or
wherein, with the thermal sensor device (1) is use, said temperature sensors (21,22) are arranged at a distance along the direction of gravitation.

13. A thermal sensor system (10) comprising the thermal sensor device (1) according to claim 11 or 12 and further comprising an absolute pressure sensor (5) or a pressure meter (6) for measuring and controlling, respectively, an absolute pressure (p) of said gas (g);
wherein said control device (4) is further configured to carry out the method according to any one of the claims 6 to 8.

14. A thermal sensor device (1) according to claim 11 or 12, or a thermal sensor system (10) according to claim 13, further comprising a thermal flow sensor device (7), wherein said control device (4) is configured to carry out the method according to claim 9 or 10.

15. A non-transitory computer readable medium (81) for thermal sensor device (1) according to claim 11 or 12 or 14, or a thermal sensor system (10) according to claim 13 or 14, said non-transitory computer readable medium (81) being encoded with a computer program product (82) comprising computer code that, when executed in a processor (84), carries out the method according to any one of the claims 1 to 10.

**Patentansprüche**

1. Verfahren zur Bestimmung eines Gasparameters (Pr;$C_H$;$H\rho$;$I_w$;$N_M$) eines Gases (g), wobei der Gasparameter (Pr;$C_H$;$H\rho$;$I_w$;$N_M$) eine Prandtl-Zahl, eine Wasserstoffkonzentration oder ein Verbrennungsparameter des Gases (g) ist, wobei das Verfahren umfasst:

- Betreiben einer thermischen Sensorvorrichtung (1), die dem Gas (g) ausgesetzt ist und mindestens einen Temperatursensor (2; 21, 22) und mindestens ein Heizelement (3) umfasst;
- Berechnen aus einer Ausgabe der thermischen Sensorvorrichtung (1):

(i) eine Temperatur (T) des Gases (g);
(ii) einen Wärmeübergangsparameter ($\lambda$) des Gases (g), wobei der Wärmeübergangsparameter ($\lambda$) des Gases (g) aus einer stationären Temperaturverteilung in dem Gas (g) bestimmt wird, die durch das mindestens eine Heizelement (3) verursacht wird;
(iii) einen wärmekapazitätsbezogenen Parameter (D;$c_p\rho$) des Gases (g), wobei der wärmekapazitätsbezogene Parameter (D; $c_p\rho$) des Gases (g) aus einem zeitlich veränderlichen Heizschema bestimmt wird, das auf das Heizelement (3) angewendet wird;
**dadurch gekennzeichnet, dass** das Verfahren ferner umfasst das Berechnen aus dem Ausgang der thermischen Sensorvorrichtung (1) von:

(iv) einem Parameter der natürlichen Konvektion $\left(\frac{1}{\nu D}\right)$ des Gases (g), der sich auf eine natürliche Konvektion bezieht, die in dem Gas (g) durch die Wirkung des mindestens einen Heizelements (3) verursacht wird; und

- Berechnen des Gasparameters (Pr;$C_H$;$H\rho$; $I_w$; $N_M$) des Gases (g) aus dem Wärmeübergangsparameter ($\lambda$),

dem wärmekapazitätsbezogenen Parameter (D; $c_p\rho$) und dem Parameter der natürlichen Konvektion $\left(\frac{1}{\nu D}\right)$.

2. Verfahren nach Anspruch 1, wobei der Parameter der natürlichen Konvektion $\left(\frac{1}{\nu D}\right)$ aus dem Wärmeübergangspa-

rameter $(\lambda)$ und der Temperatur $(T)$ des Gases $(g)$ berechnet wird, vorzugsweise durch Verwendung einer Expansion eines Ausgangs $(S_{con})$ der thermischen Sensorvorrichtung (1), der sich auf den Parameter der natürlichen Konvektion $\left(\frac{1}{\nu D}\right)$ in dem Parameter der natürlichen Konvektion $\left(\frac{1}{\nu D}\right)$ bezieht;

wobei vorzugsweise die Expansionsterme der Expansion empirisch bestimmte Funktionen des Wärmeübertragungsparameters $(\lambda)$ und der Temperatur $(T)$ des Gases $(g)$; und/oder
wobei vorzugsweise die Expansion bis zu einem linearen Term oder einem quadratischen Term durchgeführt wird.

3.   Verfahren nach einem der Ansprüche 1 bis 2, wobei die thermische Sensorvorrichtung (1) als das mindestens eine Heizelement (3) einen ersten Temperatursensor (21) und einen zweiten Temperatursensor (22) umfasst, wobei das Heizelement (3) dazwischen angeordnet ist, wobei der erste und zweite Temperatursensor (21, 22) und das Heizelement (3) vorzugsweise auf einer Brückenstruktur (11) oder einer Membran (12) angeordnet sind, die einen Hohlraum (13) in einem Substrat (14) der thermischen Sensorvorrichtung (1) überspannt;
und wobei das Verfahren umfasst:

Bestimmen eines ersten Temperaturwerts $(T_1)$ an dem ersten Temperatursensor (21) und eines zweiten Temperaturwerts $(T_2)$ am zweiten Temperatursensor (22) unter Einwirkung des Heizelements (3);
Berechnen des Wärmeübergangsparameters $(\lambda)$, des wärmekapazitätsbezogenen Parameters $(D;c_p\rho)$, des Parameters für natürliche Konvektion $\left(\frac{1}{\nu D}\right)$, und/oder des Gasparameters $(Pr;C_H;H\rho; I_W; N_M)$ unter Berücksichtigung der ersten und zweiten Temperaturwerte $(T_1, T_2)$.

4.   Verfahren nach Anspruch 3, wobei das Heizelement (3) zwischen dem ersten und dem zweiten Temperatursensor (21, 22) in einer Weise angeordnet ist, die gegenüber einem Mittelpunkt (P) zwischen den Temperatursensoren (21, 22) versetzt ist, vorzugsweise um 5 % bis 30 % eines Abstands (d) zwischen den Temperatursensoren (21, 22); und/oder
wobei die thermische Sensorvorrichtung (1) so verwendet wird, dass der erste und der zweite Temperatursensor (21, 22) entlang der Richtung der Schwerkraft getrennt sind.

5.   Verfahren nach einem der Ansprüche 1 bis 4, wobei der Wärmeübergangsparameter $(\lambda)$ eine Wärmeleitfähigkeit $(\lambda)$ und/oder der wärmekapazitätsbezogene Parameter $(D; c_p\rho)$ eine thermische Diffusivität $(D)$ oder eine volumetrische Wärmekapazität $(c_p\rho)$ des Gases $(g)$ ist.

6.   Verfahren nach einem der Ansprüche 1 bis 5, wobei zusätzlich ein Absolutdruck $(p)$ des Gases $(g)$ verwendet oder bestimmt wird und als Gasparameter ein Verbrennungsparameter $(Hp;I_w;N_M)$ des Gases $(g)$ aus dem Wärmeübergangsparameter $(\lambda)$, dem wärmekapazitätsbezogenen Parameter $(D; c_p\rho)$, dem Parameter für natürliche Konvektion $\left(\frac{1}{\nu D}\right)$ und dem Absolutdruck $(p)$ des Gases $(g)$ berechnet wird.

7.   Verfahren nach Anspruch 6, wobei der Verbrennungsparameter $(Hp;I_w;N_M)$ ausgewählt ist aus der Gruppe, die umfasst:

einen Heizwert, vorzugsweise eine Verbrennungswärme pro Volumeneinheit $(Hp)$;
einen Wobbe-Index $(I_W)$; und
eine Methanzahl $(N_M)$.

8.   Verfahren nach einem der Ansprüche 1 bis 7, wobei der Gasparameter die Prandtl-Zahl $(Pr)$ des Gases $(g)$ ist, oder wobei das Gas $(g)$ ein Erdgas ist und der Gasparameter eine Wasserstoffkonzentration $(C_H)$ des Gases $(g)$ ist.

9.   Verfahren nach Anspruch 8, wobei die thermische Sensorvorrichtung (1) ferner als ein thermischer Durchflusssensor (7) konfiguriert ist, wobei das Verfahren ferner umfasst:

(i) Einrichten eines Gasdurchflusses $(f)$ in dem Gas $(g)$;
(ii) Betreiben des thermischen Durchflusssensors (7), der dem Gasdurchfluss $(f)$ ausgesetzt ist, und Erzeugen eines Durchflusssignals $(S_{flow})$;

(iii) Einstellen eines Null-Gasdurchflusses $(f_0)$ in dem Gas (g), vorzugsweise bei konstanter Heizleistung des Heizelements (3);

(iv) Betreiben des thermischen Durchflusssensors (7), der dem Null-Gasdurchfluss $(f_0)$ ausgesetzt ist, und Erzeugen eines Null-Durchflusssignals $(S_{flow,0})$;

wobei:

(v) die Wärmeleitfähigkeit $(\lambda)$ und die thermische Diffusivität $(D)$ des Gases (g) aus dem Ausgang der thermischen Sensorvorrichtung (1) berechnet werden;

(vi) der Parameter der natürlichen Konvektion $\left(\frac{1}{vD}\right)$ basierend auf dem Null-Durchflusssignals $(S_{flow,0})$ berechnet wird;

(vii) die Prandtl-Zahl $(Pr)$ aus dem Parameter der natürlichen Konvektion $\left(\frac{1}{vD}\right)$ und der thermischen Diffusivität $(D)$ des Gases (g) berechnet wird;

und wobei:

(vii) ein Volumendurchfluss $(V)$ oder eine Durchflussgeschwindigkeit $(v)$ basierend auf dem Durchflusssignal $(S_{flow})$, der Wärmeleitfähigkeit $(\lambda)$, der thermischen Diffusivität $(D)$ und der Prandtl-Zahl $(Pr)$ des Gases (g) berechnet wird; und/oder

(viii) ein Volumendurchfluss bei Standardtemperatur (F) basierend auf dem Durchflusssignal $(S_{flow})$, der Temperatur $(T)$, der Wärmeleitfähigkeit $(\lambda)$, der thermischen Diffusivität $(D)$, und der Prandtl-Zahl $(Pr)$ des Gases (g) berechnet wird.

10. Verfahren nach Anspruch 9, wobei zusätzlich ein absoluter Druck $(p)$ des Gases (g) verwendet oder mittels eines Absolutdrucksensors (5) oder eines Druckmessers (6) bestimmt wird; wobei:

(i) ein Standard-Volumendurchfluss $(Q)$ basierend auf der Temperatur $(T)$, dem absoluten Druck (p) und dem Volumendurchfluss $(V)$ berechnet wird; und/oder

(ii) ein Energiedurchfluss $(J_E)$ basierend auf dem Verbrennungsparameter $(Hp; I_w; N_M)$ nach Anspruch 8 oder 9 und dem Volumendurchfluss $(V)$ berechnet wird.

11. Thermische Sensorvorrichtung (1) zur Bestimmung eines Gasparameters $(Pr; C_H, H\rho; I_W; N_m)$ eines Gases (g), wobei der Gasparameter $(Pr; C_H; H\rho; I_W; N_M)$ eine Prandtl-Zahl, eine Wasserstoffkonzentration oder ein Verbrennungsparameter des Gases (g) ist, wobei die thermische Sensorvorrichtung (1) umfasst:

mindestens einen Temperatursensor (2; 21, 22) und mindestens ein Heizelement (3) und dem Gas (g) ausgesetzt ist, wobei der mindestens eine Temperatursensor (2; 21, 22) und das mindestens eine Heizelement (3) vorzugsweise auf einer Brückenstruktur (11) oder auf einer Membran (12) angeordnet sind, die einen Hohlraum (13) in einem Substrat (14) der thermischen Sensorvorrichtung (1) überspannt;
eine Steuervorrichtung (4), die zur Durchführung des folgenden Verfahrens konfiguriert ist:

- Betreiben der thermischen Sensorvorrichtung (1), die dem Gas (g) ausgesetzt ist;
- Berechnen aus einem Ausgang der thermischen Sensorvorrichtung (1):

(i) eine Temperatur $(T)$ des Gases (g);
(ii) einen Wärmeübergangsparameter $(\lambda)$ des Gases (g), wobei der Wärmeübergangsparameter $(\lambda)$ des Gases (g) aus einer stationären Temperaturverteilung in dem Gas (g) bestimmt wird, die durch das mindestens eine Heizelement (3) verursacht wird;
(iii) einen wärmekapazitätsbezogenen Parameter $(D; c_p\rho)$ des Gases (g), wobei der wärmekapazitätsbezogene Parameter $(D; c_p\rho)$ des Gases (g) aus einem zeitlich veränderlichen Heizschema bestimmt wird, das auf das Heizelement (3) angewendet wird;
**dadurch gekennzeichnet, dass** die Steuervorrichtung (4) ferner so konfiguriert ist, dass sie aus dem Ausgang der thermischen Sensorvorrichtung (1) berechnet:

(iv) einen Parameter der natürlichen Konvektion $\left(\frac{1}{\nu D}\right)$ des Gases (g), der sich auf eine natürliche Konvektion bezieht, die in dem Gas (g) durch die Wirkung des mindestens einen Heizelements (3) verursacht wird; und

- Berechnen des Gasparameters $(Pr; C_H; H\rho; I_W; N_M)$ des Gases (g) aus dem Wärmeübergangsparameter $(\lambda)$, dem wärmekapazitätsbezogenen Parameter $(D; c_p\rho)$ und dem Parameter für natürliche Konvektion $\left(\frac{1}{\nu D}\right)$;

wobei vorzugsweise die Steuervorrichtung (4) ferner so konfiguriert ist, dass sie das Verfahren nach einem der Ansprüche 2 bis 5 oder 8 durchführt.

12. Thermische Sensorvorrichtung (1) nach Anspruch 11, die zwei oder mehr Temperatursensoren (21, 22) umfasst, wobei eines oder mehrere der mindestens einen Heizelemente (3) zwischen den Temperatursensoren (21, 22) und von einem Mittelpunkt (P) zwischen den Temperatursensoren (21, 22) versetzt angeordnet sind, vorzugsweise um 5 % bis 30 % eines Abstands (d) zwischen den Temperatursensoren (21, 22); und/oder wobei bei Verwendung der thermischen Sensorvorrichtung (1) die Temperatursensoren (21, 22) in einem Abstand entlang der Gravitationsrichtung angeordnet sind.

13. Thermisches Sensorsystem (10) umfassend die thermische Sensorvorrichtung (1) nach Anspruch 11 oder 12 und ferner umfassend einen Absolutdrucksensor (5) oder einen Druckmesser (6) zum Messen beziehungsweise Steuern eines Absolutdrucks (p) des Gases (g); wobei die Steuervorrichtung (4) ferner so konfiguriert ist, dass sie das Verfahren nach einem der Ansprüche 6 bis 8 durchführt.

14. Thermische Sensorvorrichtung (1) nach Anspruch 11 oder 12 oder thermisches Sensorsystem (10) nach Anspruch 13, weiter umfassend einen thermischen Durchflusssensor (7), wobei die Steuervorrichtung (4) so konfiguriert ist, dass sie das Verfahren nach Anspruch 9 oder 10 durchführt.

15. Nichtflüchtiges computerlesbares Medium (81) für eine thermische Sensorvorrichtung (1) nach Anspruch 11 oder 12 oder 14 oder ein thermisches Sensorsystem (10) nach Anspruch 13 oder 14, wobei das nichtflüchtige computerlesbare Medium (81) mit einem Computerprogrammprodukt (82) codiert ist, das einen Computercode umfasst, der, wenn er in einem Prozessor (84) ausgeführt wird, das Verfahren nach einem der Ansprüche 1 bis 10 durchführt.

## Revendications

1. Procédé de détermination d'un paramètre de gaz $(Pr; C_H; H_\rho; I_W; N_M)$ d'un gaz (g), dans lequel ledit paramètre de gaz $(Pr; C_H; H_\rho; I_w; N_M)$ est un nombre Prandtl, une concentration d'hydrogène, ou un paramètre de combustion du gaz (g), ledit procédé comprenant :

- opérer un dispositif de capteur thermique (1) étant exposé audit gaz (g) et comprenant au moins un capteur de température (2 ; 21, 22) et au moins un élément chauffant (3);
- calculer à partir d'une sortie dudit dispositif de capteur thermique (1) :

(i) une température (T) dudit gaz (g);
(ii) un paramètre de transfert de chaleur $(\lambda)$ dudit gaz (g), dans lequel ledit paramètre de transfert de chaleur $(\lambda)$ dudit gaz (g) est déterminé à partir d'une distribution de température stationnaire dans ledit gaz (g) provoquée par ledit au moins un élément chauffant (3),
(iii) un paramètre lié à la capacité calorifique $(D; c_p\rho)$ dudit gaz (g), dans lequel ledit paramètre lié à la capacité calorifique $(D; c_p\rho)$ dudit gaz (g) est déterminé à partir d'un schéma de chauffage variable dans le temps appliqué audit élément chauffant (3);
**caractérisé en ce que** le procédé comprend en outre calculer à partir de la sortie du dispositif de capteur thermique (1):
(iv) un paramètre de convection naturelle (1/νD) dudit gaz (g) lié à une convection naturelle provoquée dans ledit gaz (g) par l'action dudit au moins un élément chauffant (3) ; et

- calculer ledit paramètre de gaz *(Pr; $C_H$ ;$H_\rho$; $I_W$; $N_M$)* dudit gaz (g) à partir dudit paramètre de transfert de chaleur *($\lambda$)*, ledit paramètre lié à la capacité calorifique *(D; $c_p\rho$)*, et ledit paramètre de convection naturelle *(1/vD)*.

2. Le procédé selon la revendication 1, dans lequel ledit paramètre de convection naturelle *(1/vD)* est calculé à partir dudit paramètre de transfert de chaleur *($\lambda$)* et de ladite température *(T)* dudit gaz (g), de préférence en utilisant une expansion d'une sortie *($S_{con}$)* dudit dispositif de capteur thermique (1) lié au paramètre de convection naturelle (1/vD) dans ledit paramètre convection naturelle *(1/vD)*;

   dans lequel, de préférence, les termes d'expansion de ladite expansion sont des fonctions déterminées empiriquement dudit paramètre de transfert de chaleur ($\lambda$) et de ladite température (T) dudit gaz (g) ; et/ou
   dans lequel, de préférence, ladite expansion est constituée d'un terme linéaire ou d'un terme quadratique.

3. Le procédé selon la revendication 1 ou 2, dans lequel ledit dispositif de capteur thermique (1) comprend, comme ledit au moins un élément chauffant (3), un premier capteur de température (21) et un second capteur de température (22), ledit élément chauffant (3) étant agencé entre eux, lesdits premier et deuxième capteurs de température (21, 22) et ledit élément chauffant (3) étant de préférence agencés sur une structure de pont (11) ou une membrane (12) s'étendant sur une cavité (13) dans un substrat (14) dudit dispositif de capteur thermique (1) ;
   et dans lequel ledit procédé comprend :

   déterminer une première valeur de température *($T_1$)* au niveau dudit premier capteur de température (21) et une seconde valeur de température *($T_2$)* au niveau du second capteur de température (22) sous l'action de l'élément chauffant (3);
   calculer ledit paramètre de transfert de chaleur *($\lambda$)*, ledit paramètre lié à la capacité calorifique *(D; $c_p\rho$)*, ledit paramètre de convection naturelle (1/vD); et/ou ledit paramètre de gaz *(Pr; $C_H$ ;$H_\rho$; $I_W$; $N_M$)* en tenant compte desdites première et seconde valeurs de température *($T_1$, $T_2$)*.

4. Le procédé selon la revendication 3, dans lequel l'élément chauffant (3) est agencé entre lesdits premier et deuxième capteurs de température (21, 22) d'une manière décalée par rapport à un point médian (P) entre lesdits capteurs de température (21, 22), de préférence de 5 % à 30 % d'une distance (d) entre lesdits capteurs de température (21, 22) ; et/ou
   dans lequel le dispositif de capteur thermique (1) est utilisé de sorte que lesdits premier et deuxième capteurs de température (21, 22) sont séparés le long de la direction de la gravitation.

5. Le procédé selon l'une quelconque des revendications 1 à 4, dans lequel ledit paramètre de transfert de chaleur *($\lambda$)* est une conductivité thermique *($\lambda$)* et/ou ledit paramètre lié à la capacité calorifique *(D; $c_p\rho$)* est une diffusivité thermique *(D)* ou une capacité calorifique volumétrique *($c_p\rho$)* dudit gaz (g).

6. Le procédé selon l'une quelconque des revendications 1 à 5, dans lequel, en outre, une pression absolue *(p)* dudit gaz (g) est utilisée ou déterminée, et comme ledit paramètre de gaz, un paramètre de combustion *($H_\rho$; $I_W$; $N_M$)* dudit gaz (g) est calculé à partir dudit paramètre de transfert de chaleur *($\lambda$)*, dudit paramètre lié à la capacité calorifique *(D; $c_p\rho$)*, dudit paramètre de convection naturelle *(1/vD)* et ladite pression absolue *(p)* dudit gaz (g).

7. Le procédé selon la revendication 6, dans lequel ledit paramètre de combustion *($H_\rho$; $I_W$; $N_M$)* est choisi dans le groupe comprenant :

   une valeur calorifique, de préférence une chaleur de combustion par unité de volume *($H_p$)*;
   un indice de Wobbe *($I_W$)*; et
   un indice de méthane *($N_M$)*.

8. Le procédé selon l'une quelconque des revendications 1 à 7, dans lequel ledit paramètre de gaz est le nombre de Prandtl *(Pr)* dudit gaz (g), ou dans lequel ledit gaz (g) est un gaz naturel et ledit paramètre de gaz est une concentration d'hydrogène *($C_H$)* dudit gaz (g).

9. Le procédé selon la revendication 8, ledit dispositif de capteur thermique (1) étant en outre configuré comme un capteur de flux thermique (7), dans lequel ledit procédé comprend en outre:

   (i) établir un flux de gaz *(f)* dans ledit gaz (g) ;
   (ii) opérer ledit capteur de flux thermique (7) exposé audit flux de gaz (f) et générer un signal de flux *($S_{flow}$)* ;

EP 3 502 687 B1

(iii) établir un débit de gaz nul ($f_0$) dans ledit gaz (g), de préférence à puissance de chauffe constante vers l'élément chauffant (3) ;

(iv) opérer ledit capteur de flux thermique (7) exposé audit flux de gaz nul ($f_0$) et générer un signal de flux nul ($S_{flow,0}$) ; ; dans lequel

(v) la conductivité thermique ($\lambda$) et la diffusivité thermique (D) dudit gaz (g) sont calculées à partir de ladite sortie du dispositif de capteur thermique (1) ;

(vi) ledit paramètre de convection naturelle (1/vD) est calculé sur la base dudit signal de flux nul ($S_{flow,0}$) ;

(vii) le nombre de Prandtl (Pr) est calculé à partir dudit paramètre de convection naturelle (1/vD) et de ladite diffusivité thermique (D) dudit gaz (g) ; et dans lequel

(viii) un débit volumétrique ($\dot{V}$) ou une vitesse d'écoulement (v) est calculé sur la base dudit signal de flux ($S_{flow}$), de ladite conductivité thermique ($\lambda$), de ladite diffusivité thermique (D) et dudit nombre de Prandtl (Pr) dudit gaz (g); et/ou

(ix) un débit volumétrique à température standard (F) est calculé sur la base dudit signal de flux ($S_{flow}$) de ladite température (T), de ladite conductivité thermique ($\lambda$), de ladite diffusivité thermique (D) et dudit nombre de Prandtl (Pr) dudit gaz (g).

10. Le procédé selon la revendication 9, dans lequel, en outre, une pression absolue (p) dudit gaz (g) est utilisée ou déterminée au moyen d'un capteur de pression absolue (5) ou d'un manomètre (6) ; dans lequel:

(i) un débit volumétrique standard (Q) est calculé sur la base de ladite température (T), de ladite pression absolue (p) et dudit débit volumétrique ($\dot{V}$); et/ou

(ii) un flux d'énergie ($J_E$) est calculé sur la base dudit paramètre de combustion ($H_\rho$; $I_w$; $N_M$) selon la revendication 8 ou 9 et ledit débit volumétrique ($\dot{V}$).

11. Un dispositif de capteur thermique (1) pour déterminer un paramètre de gaz (Pr; $C_H$; $H_\rho$; $I_w$; $N_M$) d'un gaz (g), dans lequel ledit paramètre de gaz (Pr; $C_H$; $H_P$; $I_W$; $N_M$) est un nombre Prandtl, une concentration d'hydrogène, ou un paramètre de combustion du gaz (g), ledit dispositif de capteur thermique (1) comprenant :

au moins un capteur de température (2; 21, 22) et au moins un élément chauffant (3) et étant exposés audit gaz (g), ledit au moins un capteur de température (2 ; 21, 22) et ledit au moins un élément chauffant (3) étant de préférence agencés sur une structure de pont (11) ou sur une membrane (12) s'étendant sur une cavité (13) dans un substrat (14) dudit dispositif de capteur thermique (1);

un dispositif de commande (4) configuré pour effectuer le procédé suivant :

- opérer ledit dispositif de capteur thermique (1) étant exposé audit gaz (g) ;
- calculer à partir d'une sortie dudit dispositif de capteur thermique (1) :

(i) une température (T) dudit gaz (g);
(ii) un paramètre de transfert de chaleur ($\lambda$) dudit gaz (g), dans lequel ledit paramètre de transfert de chaleur ($\lambda$) dudit gaz (g) est déterminé à partir d'une distribution de température stationnaire dans ledit gaz (g) provoquée par ledit au moins un élément chauffant (3),
(iii) un paramètre lié à la capacité calorifique (D; $c_p\rho$) dudit gaz (g), dans lequel ledit paramètre lié à la capacité calorifique (D; $c_p\rho$) dudit gaz (g) est déterminé à partir d'un schéma de chauffage variable dans le temps appliqué audit élément chauffant (3);
**caractérisé en ce que** le dispositif de commande (4) est en outre configuré pour calculer à partir de la sortie dudit dispositif de capteur thermique (1):
(iv) un paramètre de convection naturelle (1/vD) dudit gaz (g) lié à une convection naturelle provoquée dans ledit gaz (g) par l'action dudit au moins un élément chauffant (3) ; et

- calculer ledit paramètre de gaz (Pr; $C_H$ ;$H_\rho$; $I_W$; $N_M$) dudit gaz (g) à partir dudit paramètre de transfert de chaleur ($\lambda$), ledit paramètre lié à la capacité calorifique (D; $c_p\rho$), et ledit paramètre de convection naturelle (1/vD) ; dans lequel, de préférence, ledit dispositif de commande (4) est en outre configuré pour effectuer le procédé selon l'une quelconque des revendications 2 à 5 ou 8.

12. Le dispositif de capteur thermique (1) selon la revendication 11, comprenant deux ou plusieurs capteurs de température (21, 22), dans lequel un ou plusieurs dudit au moins un élément chauffant (3) est agencé entre lesdits capteurs de température (21, 22) et décalé par rapport à un point médian (P) entre lesdits capteurs de température (21, 22), de préférence de 5 % à 30 % d'une distance (d) entre lesdits capteurs de température (21, 22) ; et/ou

dans lequel, lorsque le dispositif de capteur thermique (1) est utilisé, lesdits capteurs de température (21, 22) sont disposés à une distance le long de la direction de gravitation.

**13.** Un système de capteur thermique (10) comprenant le dispositif de capteur thermique (1) selon la revendication 11 ou 12 et comprenant en outre un capteur de pression absolue (5) ou un manomètre (6) pour mesurer et contrôler, respectivement, une pression absolue (p) dudit gaz (g) ;
dans lequel ledit dispositif de commande (4) est en outre configuré pour effectuer le procédé selon l'une quelconque des revendications 6 à 8.

**14.** Un dispositif de capteur thermique (1) selon la revendication 11 ou 12, ou un système de capteur thermique (10) selon la revendication 13, comprenant en outre un dispositif de capteur de flux thermique (7), dans lequel ledit dispositif de commande (4) est configuré pour effectuer le procédé selon la revendication 9 ou 10.

**15.** Un support lisible par ordinateur non transitoire (81) pour dispositif de capteur thermique (1) selon la revendication 11 ou 12 ou 14, ou un système de capteur thermique (10) selon la revendication 13 ou, ledit support lisible par ordinateur non transitoire (81) étant codé avec un produit de programme d'ordinateur (82) comprenant un code informatique qui, lorsqu'il est exécuté dans un processeur (84), effectue le procédé selon l'une quelconque des revendications 1 à 10.

$Pr, C_H, H\rho, I_W, N_M$

**FIG. 1**

**FIG. 2**

**FIG. 3**

**FIG. 4**

**FIG. 5**

Hρ from λ and D and Dv

FIG. 6

Rel. Error of Hρ from λ and D and Dv

FIG. 7

## Error of H$_2$ from $\lambda$ and D and Pr

## FIG. 8

## CH4+H2

## FIG. 9

**FIG. 10**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 10122039 A1
- EP 2015056 A
- US 20160138951 A1
- US 20100154510 A1
- US 20110126611 A1
- US 6019505 A
- WO 2015075278 A1
- EP 3153854 A1
- WO 0198736 A1
- EP 2527779 A1
- WO 2012021999 A1
- US 20040099057 A1
- EP 1840535 A
- US 5050429 A
- EP 1426740 A